# EUROPEAN PATENT APPLICATION

(11) **EP 2 273 269 A1**
(43) Date of publication of application: **12.01.2011**
(21) Application number: 09742601.9
(22) Date of filing: 23.04.2009
(51) Int. Cl.: G01N 33/543

(54) **BIOSENSOR MANUFACTURING METHOD AND BIOSENSOR**

(30) Priority: 07.05.2008 JP 2008120854
(71) Applicant: Panasonic Corporation, Kadoma-shi Osaka 571-8501 (JP)
(72) Inventor: KAWAMATA, Ryoko, Osaka 540-7207 (JP); TAKAHASHI, Mie, Osaka 540-6207 (JP)
(74) Representative: Grünecker, Kinkeldey, Stockmair & Schwanhäusser Anwaltssozietät
(86) International application number: PCT/JP2009/001859
(87) International publication number: WO 2009/136476

(57) **Abstract**

Provided are a biosensor and a method of manufacturing the same which can reduce an amount of liquid specimen required for measurement (a required amount of the liquid specimen), and conduct measurement with higher accuracy. In the biosensor, the liquid specimen is developed on a development layer by using chromatography and a test substance in the liquid specimen is measured. By reducing the thickness of the development layer (to 20 µm to 135 µm), the required amount of the liquid specimen is reduced. The thickness of the development layer is controlled to a smaller thickness thus, so that the required amount of the liquid specimen can be reduced in response to the measurement of a test substance in each liquid specimen in a state in which the accuracy of analysis is properly kept.

## Description

### Technical Field

The present invention relates to a biosensor in which a test substance in a liquid specimen is developed to a development layer and is measured thereon, and particularly relates to a biosensor and a method of manufacturing the same which can reduce an amount of liquid specimen required for measurement.

### Background Art

In recent years, home care and community health care in doctor's offices and clinics have improved and the number of early diagnoses and the number of urgent laboratory tests have increased. Against this backdrop, analyzing devices have been demanded that can quickly and easily perform measurements with high accuracy even if users are not medical technologists. Thus small analyzing devices for POCT (Point of Care Testing) have received attention that can perform reliable measurements in a short time without complicated operations.

POCT is a generic name for inspections conducted in locations "close to patients", for example, in consulting rooms of practitioners and specialists, hospitals, and clinics for outpatients. POCT has been a notable method that is quite useful for improving the quality of diagnoses such that a doctor quickly judges an inspection result, immediately performs treatment, and monitors the process of the treatment and the prognosis. Inspections conducted by such small analyzing devices can reduce the cost of transporting specimens, the cost of equipment, and the cost of unnecessary inspections, thereby reducing the total inspection cost as compared with inspections conducted in central examination rooms. In the U.S. featuring rational hospital management, the POCT market has rapidly expanded and is expected to grow worldwide, including Japan.

In a dry-type biosensor typified by an immuno-chromatographic sensor, an adjustment of a reagent is not necessary and a test substance contained in a liquid specimen such as blood and urine to be measured can be analyzed. Currently, a large number of dry-type biosensors have been put into practical use as representative POCT biosensors because dry-type biosensors are quite useful for easily and quickly analyzing a test substance in a liquid specimen. Further, in the market, it has been requested to minimize the amount of liquid specimen to enable measurement anywhere at anytime by anyone.

A typical immuno-chromatographic sensor is made up of a specimen adding portion for adding a liquid specimen, a development layer including multiple layers, and a water absorbing portion provided on the rear end of the development layer. The development layer includes a labeled reagent portion reacting with a test substance in the liquid specimen, and a reagent immobilizing portion in which a reagent specifically reacting with the test substance or a labeled reagent is immobilized. In such an immuno-chromatographic sensor, a required amount of liquid specimen is added to the specimen adding portion and the liquid specimen is developed to the development layer to start measurement. Further, the test substance in the liquid specimen reacts with the labeled reagent in the labeled reagent portion, and the reagent immobilized and retained in the reagent immobilizing portion specifically reacts with the test substance or the labeled reagent. Moreover, the test substance is qualitatively or quantitatively measured based on the signal of the reagent immobilizing portion.

In the related art, the components of an immuno-chromatographic sensor are made of absorbent materials that require a larger amount of liquid specimen than an actual development capacity. Thus measurement cannot be conducted without supplying a large amount of liquid specimen. Thus when the liquid specimen is blood, it is necessary to collect a sufficient sample amount from, e.g., an upper arm with pain. Further, when the liquid specimen is added to the specimen adding portion, generally, blood cells that are formed elements are separated from plasma by using a centrifugal separator, and then a specified amount of the liquid specimen has to be added with an instrument such as a dispenser and a pipette, so that an excessive sample amount is necessary. For this reason, methods for reducing an amount of liquid specimen have been studied.

In a method of reducing an amount of liquid specimen (a required amount of liquid specimen) required for measurement, a small amount of liquid specimen diluted with a large amount of diluent is added, resulting in complicated operations with insufficient simplicity and rapidity. In another method, a small amount of liquid specimen is added and then a large amount of developing solution is added to the liquid specimen, resulting in low simplicity and rapidity. In still another method, the overall size of a biosensor is reduced and the components (e.g., a sample adding portion and a development layer) are reduced in width and length, so that a required amount of liquid specimen decreases. However, a reduction in the widths and lengths of the components of the biosensor makes it difficult to produce the biosensor. For example, a reagent immobilizing portion to be measured is also reduced in size and manufacturing variations are likely to occur, so that the accuracy of measurement may decline.

Thus in recent years, in still another method enabling measurement with a small amount of liquid specimen in this type of biosensor, a specimen adding portion includes a clearance formed of a liquid impermeable material as described in Patent Literature 1. According to this method, a specified amount of liquid specimen is sipped into the clearance and the sipped liquid specimen is sufficiently developed to a development layer without causing a loss, thereby enabling simple measurement with high accuracy. In other words, the specimen adding portion made of an absorbent material in the related art includes the clearance formed of a liquid impermeable material. With this configuration, the added liquid specimen can be developed to the development layer without remaining in the specimen adding portion. It is therefore possible to eliminate a loss of liquid specimen in the specimen adding portion, the loss being caused by a sensor component material in the related art. Consequently, a required amount of liquid specimen can be reduced. In this case, the clearance is formed as a space for flowing the liquid specimen between the sample adding portion and the development layer and an inflow can be specified by the volume of the clearance. Thus it is possible to achieve simple measurement without complicated operations such as quantification of a constant volume.

Patent Literature 2 discloses a method in which a cell constrictor such as inorganic salt, amino acid, and sacchharide is carried. In this method, cell components are separated after a cell is constricted or a liquid specimen with mixed cell components is developed after or during the constriction of the cell. This method makes it possible to obtain a sufficient amount of liquid specimen to be developed to a development layer, achieving simple and quick measurement with high accuracy without performing pretreatment of the liquid specimen or using a developing solution. In other words, cell components in the liquid specimen are constricted in contact with the cell constrictor and the liquid specimen can be efficiently developed on a chromatographic carrier without causing clogging. In the case where the cell constrictor is retained in a clearance serving as a specimen adding portion, the liquid specimen flows into the clearance while the cell constrictor is dissolved and dispersed, thereby constricting the cell components in the liquid specimen. Thus a liquid specimen such as blood containing cell components may be directly added to the clearance without undergoing an operation such as centrifugal separation beforehand. With this method, it is possible to eliminate the need for separation of cell components and develop a liquid specimen with mixed cell components. For example, in the case of blood, it is not necessary to separate blood cells from plasma, thereby reducing the amount of liquid specimen to a half or less in measurement. However, even in the foregoing method, a reduction in the amount of liquid specimen is limited, resulting in limited response to market demand for reducing a burden on a patient by reducing the amount of liquid specimen.

### Citation list

### Patent Literature

Patent Literature 1: International Publication No. WO 01/90754
Patent Literature 2: Japanese Patent No. 3655283

### Disclosure of the Invention

### Technical Problem

In many cases, an amount of blood required in a typical immuno-chromatographic sensor exceeds an amount of blood obtained simply by puncture on a finger tip. Biosensors for POCT are required to conduct simple and quick measurement and it is desirable to painlessly collect a trace amount of sample from a part other than an upper arm, e.g., from a finger tip with a lancet (a knife for puncture or stab).

A required amount of liquid specimen can be reduced by a method disclosed in Patent Literature 1 and so on in which a loss of liquid specimen can be reduced by changing component materials and the configuration. Thus a required amount of liquid specimen can be reduced to some extent. These methods make it possible to achieve simple measurement with high accuracy even in the case of a relatively small amount of liquid specimen. As compared with chromatographic sensors of the related art, measurement can be conducted with a smaller amount of liquid specimen. When a liquid specimen is blood, measurement can be conducted with blood collected by puncture on a fingertip. Even in this method, however, a reduction in the amount of liquid specimen is limited and thus it is difficult to reduce a burden on a patient by reducing the amount of liquid specimen.

The present invention has been devised in view of the disadvantage and problem. An object of the present invention is to provide a biosensor and a method of manufacturing the same which can reduce a required amount of liquid specimen to a trace amount and conduct measurement with higher accuracy.

### Solution to Problem

In order to solve the disadvantage and problem, a method of manufacturing a biosensor according to the present invention is a method of manufacturing a biosensor in which a liquid specimen is developed to a development layer by using chromatography and a test substance in the liquid specimen is measured, wherein a required amount of the liquid specimen is reduced by reducing the thickness of the development layer.

In this case, the development layer is a passage for developing the liquid specimen, for example, a porous film or a small space formed of any member. The thickness is the height of a part where the liquid specimen is developed. For example, when the development layer is a porous film, the thickness of the porous film is used. When the development layer is a small space formed of any member typified by µTAS (micro total analysis systems) and MEMS (micro electro mechanical systems), the height of the small space is used. By reducing the thickness of the development layer, the capacity of the developed liquid specimen is reduced.

By reducing the thickness of the development layer to a smaller thickness, the volume of the development layer can be reduced and the amount of the liquid specimen for the development can be controlled. Thus it is possible to reduce a required amount of the liquid specimen in response to the measurement of a test substance in each liquid specimen.

A biosensor according to the present invention is a biosensor in which a liquid specimen is developed to a development layer by using chromatography and a test substance in the liquid specimen is measured, wherein the development layer is 20 µm to 135 µm in thickness.

In a biosensor of the related art, a development layer is about 150 µm in thickness. By reducing the thickness of the development layer, a required amount of the liquid specimen can be reduced.

The biosensor is a sensor that specifically detects a test substance contained in a liquid specimen and makes a quantitative decision, a semi-quantitative decision, and a qualitative decision by using proteins including enzymes, antigens, and antibodies and having specific reaction and biological materials including ribonucleic acids such as DNAs and RNAs.

By reducing the thickness of the development layer thus, background causing noise depending on the thickness of the development layer can be reduced. Thus an S/N ratio and the accuracy of measurement can be improved. Moreover, a required amount of the liquid specimen can be reduced without excessively reducing the width and length of the biosensor. Thus the biosensor does not become difficult to handle and reagent immobilizing portions at measurement points are not reduced in size. Consequently, the accuracy of measurement of the test substance does not decrease with the size reduction of the reagent immobilizing portions.

The biosensor according to the present invention further includes: a support that supports the development layer; a specimen adding portion for adding the liquid specimen to the development layer; a labeled reagent portion that reacts with a test substance in the liquid specimen; and reagent immobilizing portions each of which contains an immobilized reagent specifically reacting with one of the test substance and a labeled reagent.

With this configuration, a required amount of the liquid specimen can be reduced. Thus it is possible to reduce the amount of the labeled reagent reacting with the test substance in the liquid specimen and the amount of the reagent specifically reacting with one of the test substance and the labeled reagent as compared with the related art. Even when the development layer is reduced in thickness and strength, the support with a high strength makes it possible to properly keep the ease of handling of the biosensor. The development layer may have any configuration, for example, the development layer may be formed directly on the support or bonded on the support. The support may be made of any materials including paper, metals, and synthetic resins such as ABS and PET. Preferably, the support is made of a liquid impermeable material.

In the biosensor according to the present invention, the development layer is formed of at least one layer. With this configuration, it is possible to develop a liquid specimen in a state in which the reagent is securely retained in the development layer formed of at least one layer, achieving a simple and quick analysis on the liquid specimen with higher accuracy.

The biosensor of the present invention may be composed of an immuno-chromatographic sensor. The immuno-chromatographic sensor is a sensor for detecting a test substance in a liquid specimen by using an antigen-antibody reaction on a carrier (development layer) for chromatographic development. Generally, an immuno-chromatographic sensor has a large amount of liquid specimen. By reducing the thickness of a development layer, it is possible to provide an immuno-chromatographic sensor enabling measurement with a small amount of liquid specimen. Further, it is possible to achieve high accuracy preventing an erroneous decision of a user of the immuno-chromatographic sensor, and a simple operation performed anywhere at anytime by anyone.

The biosensor according to the present invention may be composed of an immuno-chromatographic sensor for one-step measurement. In the one-step measurement, when a liquid specimen is blood, the blood is collected by a lancet or a syringe and then the blood is added to the biosensor without undergoing pretreatment including centrifugal separation. When the liquid specimen is not blood, it is not necessary to perform pretreatment such as discarding of a bacteria solution. Thus even in immunoassay, it is possible to easily and quickly conduct analyses with higher accuracy without the need for complicated operations such as pretreatment and cleaning.

In the biosensor according to the present invention, the development layer may be composed of a porous film. Thus the chromatographic development of the liquid specimen containing the test substance can be automatically performed by capillarity. In the reagent immobilizing portions, the reagent can be three-dimensionally immobilized and an immobilization density is increased by using a porous film, thereby obtaining a sufficient signal for measurement. The porous film may be so permeable to a solution that the chromatographic development of the liquid specimen containing the test substance is automatically performed by capillarity and immobilize, by any method, the reagent specifically reacting with the labeled reagent. For example, cellulose films (including filter paper and nitrocellulose films), nylon films, glass fiber films, and porous plastic fabrics (including polyethylene and polypropylene) are available. A porous film mainly composed of nitrocellulose such as pure nitrocellulose and a mixture of nitrocellulose is preferably used but other materials may be used.

### Advantageous effects of Invention

According to the present invention, in a method of manufacturing a biosensor in which a liquid specimen is developed to a development layer and a test substance in the liquid specimen is measured, a required amount of the liquid specimen can be reduced by reducing the thickness of the development layer and the development layer is 20 µm to 135 µm in thickness in the biosensor in which the liquid specimen is developed to the development layer and the test substance is measured in the liquid specimen. Thus it is possible to reduce the required amount of the liquid specimen and easily and quickly analyze the test substance from a trace amount of the liquid specimen with higher accuracy.

### Brief Description of the Drawings

FIG. 1 is an exploded perspective view showing a biosensor using chromatography according to an embodiment of the present invention;
FIG. 2 is a perspective view showing the biosensor using chromatography according to the embodiment of the present invention;
FIG. 3 is a perspective view showing a signal measurement state in reagent immobilizing portions on the biosensor according to the embodiment;
FIG. 4 is a table showing required amounts of liquid specimen according to the thickness of a nitrocellulose film (development layer) in the biosensor according to the embodiment;
FIG. 5 is a graph showing absorbance results for each thickness of the nitrocellulose film in the biosensor according to the embodiment;
FIG. 6 is a graph showing the results of absorbance ratios (absorbances/the mean value of absorbances) for each thickness of the nitrocellulose film in the biosensor according to the embodiment;
FIG. 7 is a graph showing CV value results for each thickness of the nitrocellulose film in the biosensor according to the embodiment;
FIG. 8 is a graph showing the results (the mean value of absorbances in respective forms/the mean value of absorbances in the form of the related art) of absorbance ratios when the pore size and thickness of the nitrocellulose film are changed in the biosensor according to the embodiment; and
FIG. 9 is a graph showing CV value results when the pore size and thickness of the nitrocellulose film are changed in the biosensor according to the embodiment.

### Best Mode for Carrying Out the Invention

An embodiment of a biosensor according to the present invention will be specifically described below with reference to the accompanying drawings. The following embodiment is merely exemplary and the present invention is not always limited to the following embodiment.

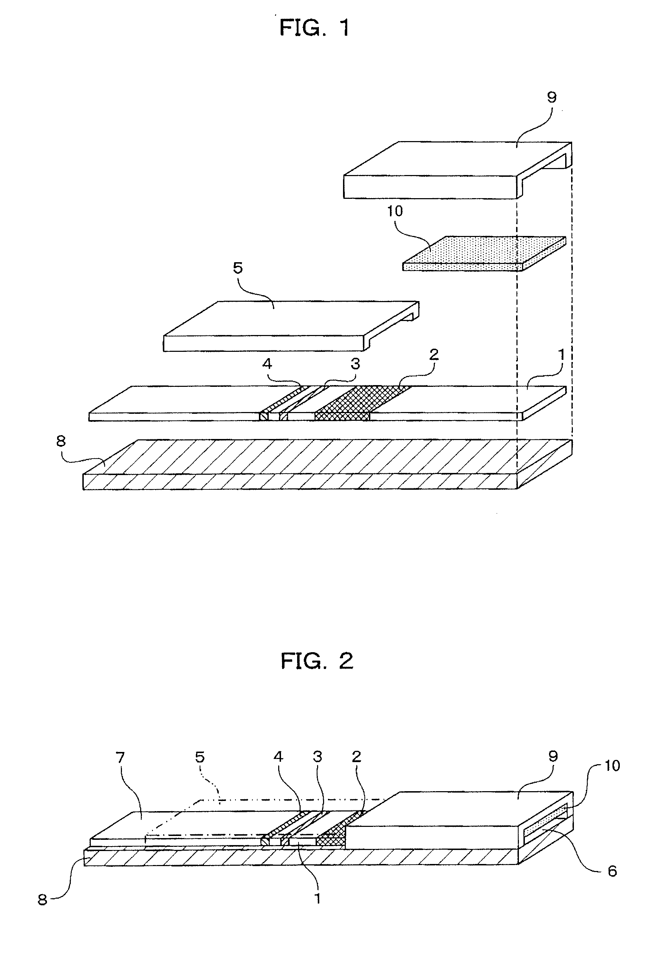

FIGS. 1 and 2 are an exploded perspective view and a perspective view showing the biosensor using chromatography according to the first embodiment of the present embodiment.

In FIG. 1, reference numeral 1 denotes a development layer in which a liquid specimen is developed. The development layer 1 is made of a material such as nitrocellulose. The material of the development layer 1 is not limited to nitrocellulose and any material such as filter paper, nonwoven fabric, membrane, fabric, and glass fiber may be used as long as a passage for developing the liquid specimen can be formed. In the present embodiment, the development layer is composed of a single layer. The single layer means a single-layer configuration and multiple layers mean a configuration in which multiple layers are arranged in parallel or in a vertical direction and the liquid specimen applied to the first layer of the multiple layers can sequentially move to the subsequent layers.

Reference numeral 2 denotes a labeled reagent portion carried on a part of the development layer 1. The labeled reagent portion 2 contains a gold-colloid labeled antibody (labeled reagent) for a test substance in the liquid specimen in a dry state in which the labeled reagent can be dissolved by the development of the liquid specimen. The labeled reagent is obtained by labeling a specific reactant such as an antibody with a marker such as a gold colloid and is used as a detector of binding in reagent immobilizing portions 3 and 4, which will be described later. The gold colloid is merely exemplary and any marker may be selected optionally when necessary. For example, metal or nonmetal colloid particles, enzymes, proteins, coloring matters, fluorescent dyes, and dye particles such as latex may be used. The labeled reagent portion 2 does not always have to be placed on the development layer 1 and may be provided at any position upstream of the reagent immobilizing portions 3 and 4, which will be described later, in the development direction of the liquid specimen.

Reference numeral 3 denotes the first reagent immobilizing portion and reference numeral 4 denotes the second reagent immobilizing portion. The first reagent immobilizing portion 3 and the second reagent immobilizing portion 4 are carried downstream of the labeled reagent portion 2 in the development direction of the liquid specimen on the development layer 1. Reagents used in the first and second reagent immobilizing portions 3 and 4 are, e.g., antibodies capable of specifically reacting with a test substance in the liquid specimen. For example, in the case of antibodies, the antibodies are bound with epitopes different from the labeled reagent and are immobilized in a dry state. The antibody used for the first reagent immobilizing portion 3 and the antibody used for the second reagent immobilizing portion 4 have different affinities for the test substance in the liquid specimen.

Any antibodies may be used for the first and second reagent immobilizing portions 3 and 4 as long as a complex of the labeled reagent and the test substance can be formed. Thus the epitopes and affinities for the test substance may be the same or different from each other. Alternatively, the two antibodies may have different affinities but have the same epitope.

In FIG. 1, the reagent immobilizing portions are provided at two points. The reagent immobilizing portions do not always have to be located at two points. At least one point can be freely selected depending on the purpose. Further, the shape on the development layer 1 does not always have to be linear and the reagent immobilizing portion may be freely shaped like a spot, a character, or a key. In FIG. 1, the first reagent immobilizing portion 3 and the second reagent immobilizing portion 4 are separated from each other but do not always have to be separated from each other. The reagent immobilizing portions may be in contact with each other like a single line.

Reference numeral 5 denotes a liquid impermeable sheet material that is composed of transparent PET tape. The liquid impermeable sheet material 5 tightly covers
the development layer 1 other than a portion connecting to a small space serving as a specimen adding portion 6 and the rear end for receiving the liquid specimen.

The development layer 1 is covered with the liquid impermeable sheet material 5 thus, so that a portion outside the specimen adding portion 6 can be protected from dropping and contamination from the outside can be prevented. Further, it is possible to prevent the developing liquid specimen from evaporating during the development of the liquid specimen, surely pass the liquid specimen through the reagent immobilizing portions 3 and 4 and the labeled reagent portion 2 that serve as reaction portions on the development layer 1, and allow the reaction portions to efficiently react with the test substance in the liquid specimen. In this case, the contamination from the outside means that the liquid specimen accidentally comes into contact with the reaction portions on the development layer 1 or an examinee directly touches the development layer 1 by hand or the like. The liquid impermeable sheet material 5 covering the development layer 1 is preferably made of transparent materials because the coloring of the first reagent immobilizing portion 3 and the second reagent immobilizing portion 4 is measured using reflected light, scattered light, or transmitted light. A portion covering the reagent immobilizing portions 3 and 4 is a signal measuring portion and thus has to be kept at least in a transmissive state.

For measurement with higher accuracy, the top surface of the development layer 1 may be tightly sealed particularly including the labeled reagent portion 2 and the reagent immobilizing portions 3 and 4, and the sides of the development layer 1 may be tightly sealed in a similar manner in parallel with the developing direction of the liquid specimen.

Reference numeral 7 denotes an opened portion of the development layer 1. Reference numeral 8 denotes a substrate supporting the development layer 1. The substrate 8 is composed of a liquid impermeable sheet material such as a PET film. The liquid impermeable sheet material forming the substrate 8 may be any material, e.g., synthetic resin materials such as ABS, polystyrene, and polyvinyl chloride, a metal, or glass in addition to a PET film.

The substrate 8 reinforces the development layer 1 and blocks an infectious specimen such as blood, saliva, and urine. When the development layer 1 gets wet with optical transparency, the substrate 8 may have the effect of blocking light. The substrate 8 may be transparent, translucent, or opaque. Any material may be used depending on the purpose. For example, a transparent material is used when the degree of reaction is optically measured through transmitted light, and an opaque material is used for the measurement of reflected light.

Reference numeral 9 denotes a small space forming material that forms a space where the liquid specimen is supplied by capillarity, that is, a small space serving as the specimen adding portion 6. The small space forming material 9 is composed of stacked transparent PET films. The small space forming material 9 provides protection against contamination by the liquid specimen to the outside when the biosensor is handled after the liquid specimen is added. The small space forming material 9 may be synthetic resin materials such as ABS, polystyrene, and polyvinyl chloride, and liquid permeable materials such as a metal and glass. The small space forming material 9 is preferably transparent or translucent but may be colored. Further, any opaque material may be used.

The specimen adding portion 6 is a small space formed by the small space forming material 9. The specimen adding portion 6 can receive the liquid specimen by capillarity. Further, the specimen adding portion 6 is connected to the development layer 1. By applying the liquid specimen into the specimen adding portion 6, the liquid specimen can start developing to the development layer 1.

Referring to FIGS. 1 and 2, the following will describe a method of measuring a test substance in the liquid specimen by using the biosensor of the present embodiment.

By bringing the liquid specimen into contact with the specimen adding portion 6, the liquid specimen naturally flows into the specimen adding portion 6 by capillarity without any mechanical operations, and flows and develops on the development layer 1. Whether the inflow of the liquid specimen is sufficient or not can be confirmed through the small space forming material 9. When it is necessary to add a certain amount of liquid specimen, the amount to be added can be accurately specified by keeping constant the volume of the small space of the specimen adding portion 6. Further, more than a certain amount of the liquid specimen can be obtained by keeping a volume larger than the required amount.

In the specimen adding portion 6, a cell component constrictor 10 is provided. The cell component constrictor 10 should be provided when the liquid specimen contains cell components, but the cell component constrictor 10 is not particularly necessary for a liquid specimen containing no cell components. The cell component constrictor 10 may be potassium chloride or sodium chloride that can constrict cells, an inorganic compound containing sodium phosphate salt, amino acids such as glycine and glutamic acids, imino acids such as proline, saccharides such as glucose, sucrose, and trehalose, and sugar alcohols such as glucitols. A system including the cell component constrictor 10 is particularly effective when whole blood is used as a liquid specimen.

The liquid specimen added into the specimen adding portion 6 develops to the development layer 1 from a contact portion between the specimen adding portion 6 and the development layer 1. When the liquid specimen reaches the labeled reagent portion 2, the labeled reagent starts dissolving. When the liquid specimen contains a test substance, the liquid specimen develops while the labeled reagent reacts with the test substance, and then the liquid specimen reaches the first reagent immobilizing portion 3. When the liquid specimen contains the test substance, a complex of the antibody immobilized in the first reagent immobilizing portion 3, the test substance in the liquid specimen, and the labeled reagent is formed according to the amount of the test substance.

Next, the liquid specimen reaches the second reagent immobilizing portion 4. When the liquid specimen contains the test substance, a complex of the antibody immobilized in the second reagent immobilizing portion 4, the test substance, and the labeled reagent is formed according to the amount of the test substance.

After reaching the second reagent immobilizing portion 4, the liquid specimen reaches the opened portion 7 of the development layer 1. The opened portion 7 is opened without being covered with the liquid impermeable sheet 5, so that the liquid specimen volatilizes or evaporates when or after reaching the opened portion 7. Further, the liquid specimen exudes to the opened portion 7 and the liquid specimen only on the opened portion 7 of the development layer 1 reaches the same level or substantially the same level as the liquid specimen in the small space of the specimen adding portion 6 on the development layer 1. Generally, a water absorbing portion is provided instead of the opened portion 7. By using a porous material with higher water retention and water absorption for the development layer 1, it is possible to pass the liquid specimen on the development layer 1 while absorbing or aspirating the liquid specimen, and shorten the measurement time. The opened portion 7 is provided with a similar effect. Particularly, a technique using the specimen adding portion 6 or the opened portion 7 is suitable for a small amount of liquid specimen, for example, when a liquid specimen is blood collected by puncture on a fingertip.

The measured value of the test substance in the liquid specimen is obtained by measuring signals from the labeled reagents of the first reagent immobilizing portion 3 and the second reagent immobilizing portion 4.

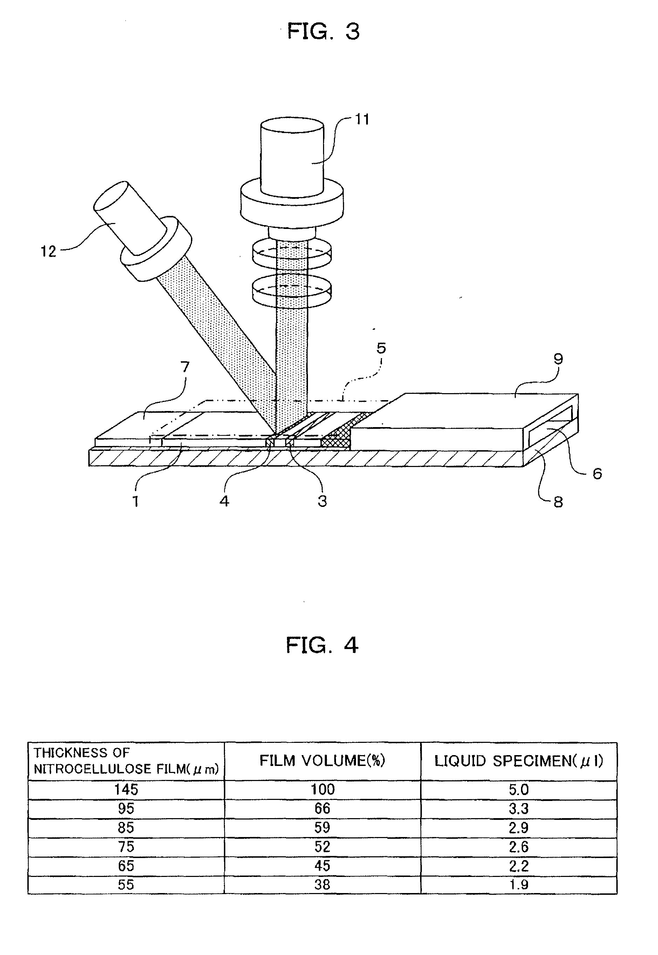

FIG. 3 shows a signal measuring unit in a state in which the signals from the labeled reagents of the reagent immobilizing portions 3 and 4 are measured on the biosensor. Reference numeral 11 denotes a light emitter for irradiating the development layer 1 with light in an analyzing device that measures and analyzes the concentration of the test substance in the liquid specimen by using the biosensor. Reference numeral 12 denotes a light receiver for detecting an optical change of the reflected light or transmitted light of light emitted from the light emitter 11 to the development layer 1. The signal measuring unit measures the signals from the labeled reagents of the reagent immobilizing portions 3 and 4 by using the light emitter 11 and the light receiver 12. The signals rise in the reagent immobilizing portions 3 and 4 as compared with the other portions and the signal strengths fluctuate with the degree of coloring of the reagent immobilizing portions 3 and 4.

The light emitter 11 is preferably a visible region or a near-visible region. An LED (Light Emitting Diode) or an LD (Laser Diode) can be selected as needed.

Further, the signals measured from the labeled reagents of the reagent immobilizing portions 3 and 4 by the signal measuring unit (the light emitter 11 and the light receiver 12) are subjected to arithmetic processing, so that the measured value of the test substance can be determined. The measured value of the test substance at that time is a test substance value that is determined,
by using a calibration curve, from the signals obtained from the labeled reagents by the signal measuring unit. The calibration curve is a regression equation representing the relationship between the signals obtained by the signal measuring unit and the value of the test substance in the liquid specimen. When a liquid specimen containing an unknown amount of test substance is measured, the measured value of the test substance in the unknown liquid specimen can be calculated by substituting the signals obtained by the signal measuring unit. The signals may be measured by any method such as reading of an optical change, an electrical change, or an electromagnetic change and acquisition of an image.

The reaction example described a sandwich reaction in which an antigen-antibody reaction for detecting an antigen by using an antibody is utilized for an immobilized reagent and a labeled reagent. A reaction system with a competitive reaction may be used by selecting a reagent.

In the case where a specific reaction is used, measurement with a reaction other than an antigen-antibody reaction can be conducted by the reagent components of a system for forming any reaction on the biosensor. Examples of a combination of a specific substance for a specific reaction and a specific binding substance for the specific substance include: antigens and antibodies against the antigens, complementary nucleotide sequences, effector molecules and receptor molecules, enzymes and inhibitors, enzymes and cofactors, enzymes and substrates, compounds containing sugar chains and lectins, antibodies and antibodies against the antibodies, receptor molecules and antibodies against the receptor molecules, substances chemically modified without eliminating specific binding activity, or a reaction system using a composite material bound to other components.

Methods for implementing the present invention will be more specifically described in accordance with the following examples. The present invention is not limited to the following examples.

### (Example 1)

### (The quantification of whole blood CRP when a development layer is reduced in thickness)

An immuno-chromatographic sensor was produced that was a biosensor including a first reagent immobilizing portion 3 in which anti-CRP antibody A was immobilized, a second reagent immobilizing portion 4 in which anti-CRP antibody B was immobilized, and a labeled reagent portion 2 containing a complex (labeled reagent) of anti-CRP antibody C and a gold colloid, on a development layer 1 including a nitrocellulose film. The immuno-chromatographic sensor is shown in FIGS. 1 and 2. In FIGS. 1 and 2, the immuno-chromatographic sensor includes the first reagent immobilizing portion 3 and the second reagent immobilizing portion 4 in which the antibodies are immobilized, the labeled reagent portion 2 that is provided between the reagent immobilizing portions and the adding portion of a liquid specimen and contains a complex of anti-CRP antibody C and a gold colloid, and a specimen adding portion 6.

The immuno-chromatographic sensor was produced as follows:

### a) Preparation of the immuno-chromatographic sensor

A solution of anti-CRP antibody A was prepared. The concentration of the solution was adjusted by diluting the solution with a phosphate buffer. The antibody solution was applied on the nitrocellulose film (development layer 1) by using a solution dispenser, so that an immobilized antibody line serving as the first reagent immobilizing portion 3 was obtained on the nitrocellulose film.

Similarly, a solution of anti-CRP antibody B was applied 2 mm downstream of the specimen adding portion. The thickness of the nitrocellulose film was varied from 145 µm to 95 µm, 85 µm, 75 µm, 65 µm, and 55 µm. After the nitrocellulose film was dried, the film was immersed into a Tris-HCl buffer solution containing 1% of skim milk and was gently swung in the buffer solution for thirty minutes. After thirty minutes, the film was moved into the bath of the Tris-HCl buffer solution and then was gently swung for ten minutes. After that, the film was gently swung in another bath of the Tris-HCl buffer solution for ten minutes and then the film was cleaned. Next, the film was immersed into a Tris-HCl buffer solution containing 0.05% of sucrose monolaurate and was gently swung for ten minutes. After that, the nitrocellulose film was removed from the bath and then was dried at room temperature. Thus the first immobilized antibody line serving as the first reagent immobilizing portion 3 and an immobilized antibody line serving as the second reagent immobilizing portion 4 were obtained on the nitrocellulose film (development layer 1).

The gold colloid was prepared by adding a solution containing 1% of trisodium citrate to a solution containing 0.01% of refluxing chloroauric acid at 100°C. After refluxing for 15 minutes, the solution was cooled at room temperature. Anti-CRP antibody C was added to the gold colloid solution having been prepared to pH 8.9 by 0.2 M of a potassium carbonate solution, and then the solution was stirred for several minutes. After that, a solution containing 10% of BSA (bovine serum albumin) of pH 8.9 was added to the solution such that the final amount of the BSA solution was 1%. The antibody-gold colloid complex (labeled reagent) was prepared thus. The labeled reagent solution was centrifugally separated at 4°C and 20000 G for 50 minutes, so that the labeled reagent was isolated. The labeled reagent was suspended in a cleaning buffer solution (1% BSA 5% sucrose-phosphoric acid buffer solution) and then was subjected to the centrifugal separation, so that the labeled reagent was cleaned and isolated. The labeled reagent was suspended by a cleaning buffer solution and was filtered by a 0.8-µm filter. After that, the labeled reagent solution was prepared to have an absorbance of 150 at 520 nm and was stored at 4°C. The labeled reagent solution was set in a solution dispenser and was applied from a point separated from the first immobilizing line (the first reagent immobilizing portion 3) and the second immobilizing line (the second reagent immobilizing portion 4) on the dried film where the immobilized anti-CRP antibody A and the immobilized anti-CRP antibody B were applied. The labeled reagent solution was applied such that the labeled reagent, the first immobilizing line, and the second immobilizing line were sequentially arranged in the addition starting direction of the specimen adding portion 6. After that, the film was subjected to vacuum freeze-drying. Thus a reaction layer carrier was obtained that included the reagent immobilizing portions 3 and 4 and the labeled reagent portion 2.

Next, the reaction layer carrier containing the prepared labeled reagent was bonded on a substrate 8 made of white PET with a thickness of 0.5 mm, and transparent tape serving as a liquid impermeable sheet material 5 was bonded from the labeled reagent portion 2 to the rear end. After that, the substrate was cut to a width of 2.0 mm with laser. After the cutting, a small space forming material 9 fabricated by stacking transparent PET with a thickness of 100 µm was bonded on the starting end where the transparent tape was not bonded, thereby forming a specimen adding portion 6 including a small space (2.0 mm in width x 7.0 mm in length x 0.3 mm in height). The space forming material 9 was immediately frozen by liquid nitrogen after dropping of a solution containing 10% of potassium chloride, and then the space forming material 9 was subjected to freeze-drying. Thus the small space forming material 9 was produced in which a cell component constrictor 10 containing dried potassium chloride was retained. The immuno-chromatographic sensor was produced thus.

### b) Preparation of a liquid specimen

A CRP solution with a known concentration was added to human blood to which heparin had been added as an anticoagulant, so that blood with CRP concentrations (test substance concentrations) of 0.03 mg/dL, 0.1 mg/dL, 0.3 mg/dL, and 1 mg/dL was prepared as liquid specimens.

### c) Measurement of the degree of coloring on the immuno-chromatographic sensor

By changing the thickness of the nitrocellulose film serving as the development layer 1 from 145 µm, which is a standard thickness of the related art, to 100 µm or less, the nitrocellulose film (development layer 1) is reduced in thickness by at least about 30%. The capacity decreases with a reduction in thickness. Thus as a matter of course, a required amount of liquid specimen for measurement can be reduced by at least 30%. FIG. 4 shows required amounts of liquid specimen for the respective thicknesses of the nitrocellulose film. For example, a nitrocellulose film having a thickness of 145 µm according to the related art requires 5 µL of liquid specimen, whereas a nitrocellulose film having a thickness of 55 µm allows even 2 µL of liquid specimen to sufficiently reach the positions of the immobilized antibody lines serving as the reagent immobilizing portions 3 and 4, enabling measurement with a trace amount of liquid specimen.

To the specimen adding portion 6 of the immuno-chromatographic sensor, a proper amount of whole blood was added that contained CRP having been prepared in step b). Further, the specimen was developed to the opened portion 7 and underwent an antigen-antibody reaction. The coloring states of the reagent immobilizing portions 3 and 4 were measured five minutes after the addition of the sample to the immuno-chromatographic sensor. In other words, as shown in FIG. 3, the immuno-chromatographic sensor was scanned by a light emitter 11 including a semiconductor laser light source of 635 nm and a light receiver 12 including a light receiving device (photodiode), and the amounts of binding of the labeled reagents in the first immobilizing portion 3 and the second immobilizing portion 4 were obtained as absorbances by calculating light reflected and scattered from the development layer 1. In this case, the reagent immobilizing portions 3 and 4 were provided at two points and an antibody having a high affinity was used upstream of the specimen adding portion 6. Further, the light source (light emitter 11) and the light receiving device (light receiver 12) were fixed, the immuno-chromatographic sensor was scanned while being moved, and the peak value (reflection absorbance) was read from obtained waveforms. To obtain such waveforms, the immuno-chromatographic sensor may be fixed and the light source and the light receiving device may be moved to scan the immuno-chromatographic sensor.

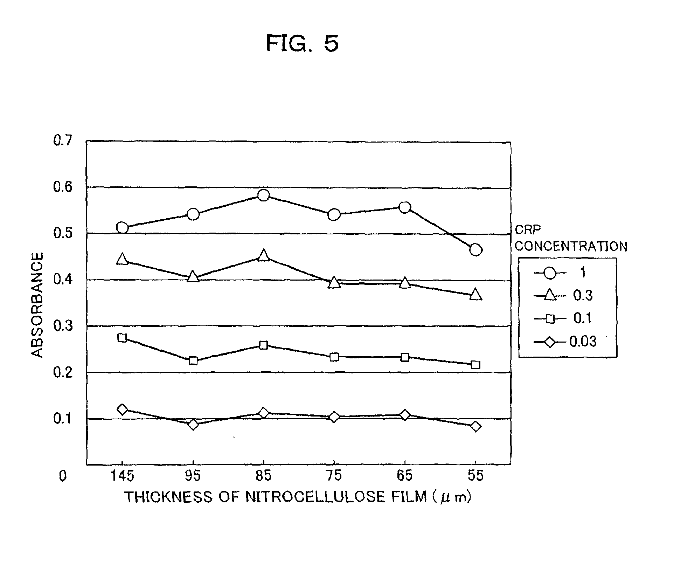

Whole blood containing the CRP prepared in step b) was measured at each CRP concentration by five immuno-chromatographic sensors (N = 5 where N is the number of immuno-chromatographic sensors). FIG. 5 shows absorbance results for each thickness of the nitrocellulose film. The horizontal axis represents a thickness of the nitrocellulose film and the vertical axis represents absorbance mean values plotted for the respective concentrations of CRP in measurement conducted at N = 5. At each CRP concentration, the absorbance does not fluctuate with the thickness of the nitrocellulose film. As shown in FIG. 5, even when the development layer 1 including the nitrocellulose film is reduced in thickness, a sufficient absorbance for quantitative measurement can be obtained. Thus it is understood that even when the nitrocellulose film is reduced in thickness, the amount of liquid specimen can be reduced without causing problems in quantitative measurement.

In the method of manufacturing the biosensor in which the liquid specimen is developed on the development layer 1 and a test substance in the liquid specimen is measured, a required amount of the liquid specimen can be reduced for the measurement of a test substance in each liquid specimen by reducing the thickness of the development layer 1. To be specific, the thickness of the development layer 1 in the biosensor is reduced to 20 µm to 135 µm from the thickness of the related art (around 150 µm, e.g., 145 µm in thickness), so that a required amount of the liquid specimen can be reduced from that of the related art.

By reducing the thickness of the development layer 1, background causing noise depending on the thickness of the development layer 1 can be reduced. Thus an S/N ratio and the accuracy of measurement can be improved. Moreover, a required amount of the liquid specimen can be reduced without excessively reducing the width and length of the biosensor. Thus the biosensor does not become difficult to handle and the reagent immobilizing portions 3 and 4 are not reduced in size. Consequently, the accuracy of measurement of the test substance does not decrease with the size reduction of the reagent immobilizing portions 3 and 4.

By reducing a required amount of the liquid specimen, it is possible to reduce the amount of the labeled reagent reacting with the test substance in the liquid specimen and the amount of the reagent specifically reacting with the test substance or the labeled reagent, thereby reducing the manufacturing cost.

### (Example 2)

### (The quantification of whole blood CRP when a development layer is reduced in thickness)

An immuno-chromatographic sensor was produced that was a biosensor including a first reagent immobilizing portion 3 in which anti-CRP antibody A was immobilized, a second reagent immobilizing portion 4 in which anti-CRP antibody B was immobilized, and a labeled reagent portion 2 containing a complex (labeled reagent) of anti-CRP antibody C and a gold colloid, on a development layer 1 including a nitrocellulose film. The immuno-chromatographic sensor is shown in FIGS. 1 and 2. In FIGS. 1 and 2, the immuno-chromatographic sensor includes the first reagent immobilizing portion 3 and the second reagent immobilizing portion 4 in which the antibodies are immobilized, the labeled reagent portion 2 that is provided between the reagent immobilizing portions and the adding portion of a liquid specimen and contains a complex of anti-CRP antibody C and a gold colloid, and a specimen adding portion 6.

The immuno-chromatographic sensor was produced as follows:

### a) Preparation of the immuno-chromatographic sensor

The thickness of a nitrocellulose film was varied from 145 µm to 95 µm, 80 µm, and 65 µm, The immuno-chromatographic sensor was produced by the same manufacturing method as in the first example and measurement was conducted as follows:

### b) Preparation of a liquid specimen

A CRP solution with a known concentration was added to human blood to which heparin had been added as an anticoagulant, so that blood with a CRP concentration (test substance concentration) of 0.1 mg/dL and hematocrit values of 20%, 30%, 40%, and 50% was prepared as a liquid specimen.

### c) Measurement of the degree of coloring on the immuno-chromatographic sensor

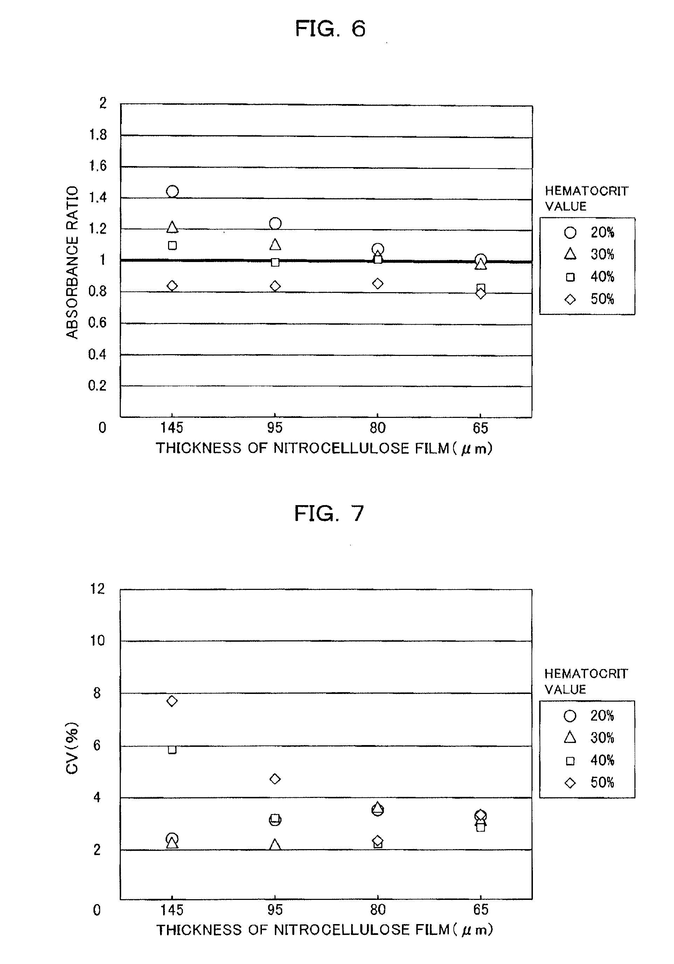

To the specimen adding portion 6 of the immuno-chromatographic sensor, a proper amount of whole blood was added that contained CRP having been prepared in step b), was developed to an opened portion 7, and underwent an antigen-antibody reaction. The coloring states of the reagent immobilizing portions 3 and 4 were measured five minutes after the addition of the sample to the immuno-chromatographic sensor. Measurements were conducted for the respective hematocrit values by five immuno-chromatographic sensors (N = 5 where N is the number of immuno-chromatographic sensors). FIG. 6 shows the results of absorbance ratios for each thickness of the nitrocellulose film. The horizontal axis represents a thickness of the nitrocellulose film and the vertical axis represents absorbance ratios plotted for the respective hematocrit values. An absorbance ratio represents a deviation of an absorbance at each hematocrit value from the mean value of absorbances at all the hematocrit values. Thus it is possible to confirm the influence of a thickness of the nitrocellulose film and a difference in hematocrit value on the measurement results. The absorbance ratios were calculated by ("the mean value of absorbances in measurement at N = 5 at each hematocrit value"/"the mean value of absorbances at all the hematocrit values"). When the film thickness was 145 µm, the absorbance ratios at the respective hematocrit values are considerably different from one another, proving that the absorbance ratios were greatly affected by the hematocrit values. Variations in absorbance ratio were reduced by reducing the film thickness. Moreover, the absorbance ratio does not greatly fluctuate with a difference in film thickness. As shown in FIG. 6, even when the development layer 1 including the nitrocellulose film is reduced in thickness, a sufficient absorbance for quantitative measurement can be obtained. It is understood that by reducing the thickness of the nitrocellulose film, it is possible to reduce a required amount of the liquid specimen and the influence of the hematocrit values, enabling quantitative measurement with high accuracy. A required amount of the liquid specimen can be adjusted by controlling the film thickness.

FIG. 7 shows CV value results for each thickness of the nitrocellulose film. The horizontal axis represents a thickness of the nitrocellulose film and the vertical axis represents CV values plotted for the respective hematocrit values in measurement conducted at N = 5. When the nitrocellulose film was 145 µm in thickness, the CV values were not satisfactory. Particularly, the CV values remarkably deteriorated at high hematocrit values. By reducing the film thickness, the influence of the hematocrit values declined and all the CV values were made satisfactory. According to the results, background causing noise depending on the thickness of the development layer 1 can be reduced by reducing the thickness of the development layer 1. Thus an S/N ratio and the accuracy of measurement can be improved. As the nitrocellulose film decreases in thickness, liquid specimen characteristics such as hematocrit values hardly cause differences in the variations of the developing speed of the liquid specimen. Thus the nitrocellulose film reduced in thickness is hardly affected by the hematocrit values as compared with a film thickness of 145 µm, so that variations in the degree of coloring were reduced and the CV values were improved. Consequently, even when the amount of the liquid specimen was reduced, the sensor was increased in sensitivity and accuracy.

### (Example 3)

### (The quantification of whole blood CRP when the thickness and the pore size of a development layer are changed)

An immuno-chromatographic sensor was produced that was a biosensor including a first reagent immobilizing portion 3 in which anti-CRP antibody A was immobilized, a second reagent immobilizing portion 4 in which anti-CRP antibody B was immobilized, and a labeled reagent portion 2 containing a complex (labeled reagent) of anti-CRP antibody C and a gold colloid, on a development layer 1 including a nitrocellulose film. The immuno-chromatographic sensor is shown in FIGS. 1 and 2. In FIGS. 1 and 2, the immuno-chromatographic sensor includes the first reagent immobilizing portion 3 and the second reagent immobilizing portion 4 in which the antibodies are immobilized, the labeled reagent portion 2 that is provided between the reagent immobilizing portions and the adding portion of a liquid specimen and contains a complex of anti-CRP antibody C and a gold colloid, and a specimen adding portion 6. The immuno-chromatographic sensor was produced as follows:

### a) Preparation of the immuno-chromatographic sensor

Sixteen forms of nitrocellulose films, which were porous carriers, were prepared with a thickness of 145 µm and a pore size of 5 µm according a form of the related art, a thickness of 135 µm and pore sizes of 3 µm, 5 µm, and 8 µm, a thickness of 115 µm and pore sizes of 3 µm and 5 µm, a thickness of 95 µm and pore sizes of 3 µm, 5 µm, 8 µm, and 10 µm, a thickness of 80 µm and pore sizes of 5 µm, 8 µm, and 10 µm, and a thickness of 60 µm and pore sizes of 5 µm, 8 µm, and 10 µm. The immuno-chromatographic sensor was produced by the same manufacturing method as in example 1 and measurements were conducted as follows:

### b) Preparation of a liquid specimen

A CRP solution with a known concentration was added to human blood to which heparin had been added as an anticoagulant, so that blood with a CRP concentration (test substance concentration) of 0.3 mg/dL was prepared as a liquid specimen.

### c) Measurement of the degree of coloring on the immuno-chromatographic sensor

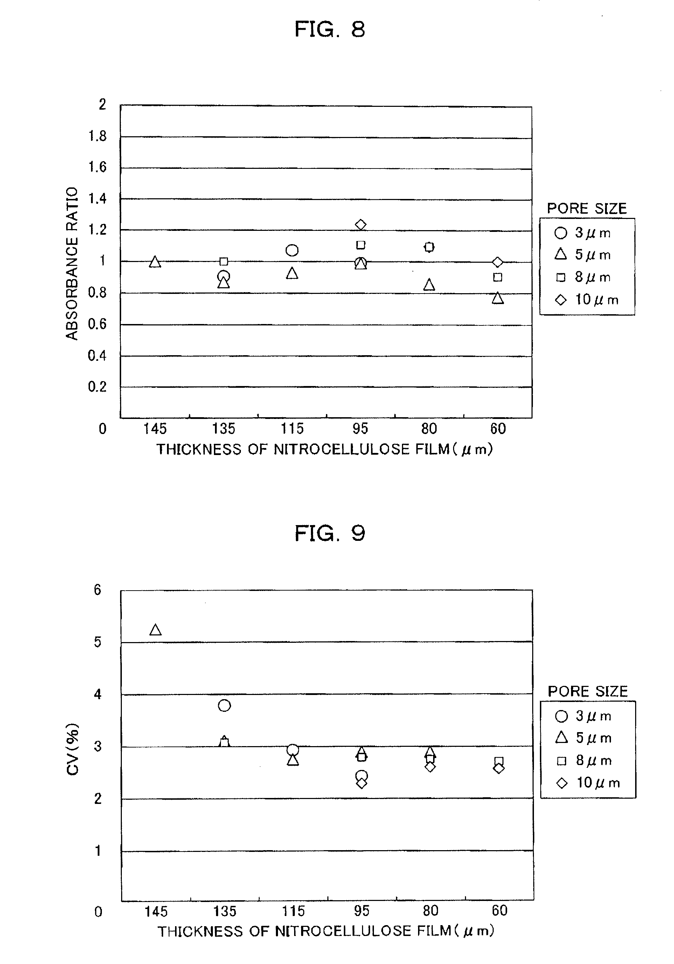

To the specimen adding portion 6 of the immuno-chromatographic sensor, a proper amount of whole blood was added that contained CRP having been prepared in step b). Further, the specimen was developed to an opened portion 7 and underwent an antigen-antibody reaction. The coloring states of the reagent immobilizing portions 3 and 4 were measured five minutes after the addition of the sample to the immuno-chromatographic sensor. The measurements were conducted by five immuno-chromatographic sensors (N = 5 where N is the number of immuno-chromatographic sensors) according to the forms of the nitrocellulose film. FIG. 8 shows the results of absorbance ratios when the thickness and the pore size of the nitrocellulose film are changed. The horizontal axis represents a thickness of the nitrocellulose film and the vertical axis represents absorbance ratios plotted for the respective pore sizes. An absorbance ratio represents a deviation of an absorbance in the forms of the nitrocellulose film from an absorbance at 145 µm in the form of the related art. Thus it is possible to confirm the influence of a thickness of the nitrocellulose film and a difference in pore size on the measurement results. The absorbance ratios were calculated by ("the mean value of absorbances in measurement at N = 5 in the forms of the nitrocellulose film"/"the mean value of absorbances in measurement at N = 5 at 145 µm in the form of the related art"). As shown in FIG. 8, an absorbance does not fluctuate with a difference in the thickness or a difference in the pore size of the nitrocellulose film. As shown in FIG. 8, even when the development layer 1 including the nitrocellulose film varies in thickness and pore size, a sufficient absorbance for quantitative measurement can be obtained.

FIG. 9 shows CV value results in each form of the nitrocellulose film. The horizontal axis represents a thickness of the nitrocellulose film and the vertical axis represents CV values plotted for the respective pore sizes in measurement conducted at N = 5 where N is the number of immuno-chromatographic sensors. When the nitrocellulose film was 145 µm in thickness in the form of the related art, the CV values were improved by reducing the thickness. Particularly, the CV values were remarkably improved at 115 µm or less. According to these results, background values causing noise depending on the thickness of the development layer 1 can be reduced by reducing the thickness of the development layer 1. Thus an S/N ratio and the accuracy of measurement were improved and variations in the degree of coloring were reduced, so that the CV values were improved. As shown in FIGS. 8 and 9, the biosensor was improved in sensitivity and accuracy by reducing the thickness of the development layer 1 regardless of the pore size of the nitrocellulose film.
A pore size in this example indicates the mean value of the pore sizes of the nitrocellulose film. The pore sizes were measured based on a cross-sectional image of the nitrocellulose film observed by a scanning electron microscope. The method of measuring pore sizes is not particularly limited and pore sizes may be measured by any method, e.g., a valve point method or a mercury pressure method. Further, a porous carrier of nitrocellulose is not composed of pores of uniform pore size. The pores are varied in size relative to the mean pore size. For specific explanation, a comparative example was described based on the mean pore size. The pore size values are not limited to the described numeric values.

The foregoing examples described the measurement examples of CRP concentrations in blood. The liquid specimens to be measured include, for example, water, solutions, body fluids such as urine, plasma, serum, and saliva, and solutions in which a solid, powder, or gas is dissolved. The liquid specimens are used for, for example, a blood test, a urinalysis, a water examination, a fecal examination, a soil analysis, and a food analysis. Further, the test substances include antibodies, immunoglobulins, hormones, proteins such as enzymes and peptides, protein derivatives, bacteria, viruses, funguses, mycoplasmas, parasites, infectious substances including products and components thereof, therapeutic agents, drugs such as drugs of abuse, and tumor markers. Specifically, the test substances may include human chorionic gonadotropin (HCG), luteinizing hormones (LH), thyroid-stimulating hormones, follicle stimulating hormones, parathyroid stimulating hormones, adrenocorticotropic hormones, estradiols, prostate specific antigens, hepatitis, myoglobins, CRPs, cardiac troponins, BNPs, HBA1Cs, and albumins. Further, the present invention can be implemented for environmental analyses such as water examinations and soil analyses, food analyses, and so on. The present invention makes it possible to easily and quickly conduct measurement with high sensitivity and high performance and enable measurement anywhere at anytime by anyone. Thus the present invention is usable as an analyzing device for POCT.

### Industrial Applicability

A biosensor according to the present invention can be used for measurement easily and quickly conducted with high sensitivity and high performance, for example, when a test substance in a small amount of liquid specimen is detected and analyzed for quantification and semi-quantification based on any reaction such as an antigen-antibody reaction. Further, the biosensor enables measurement anywhere at anytime by anyone. Thus the present invention is useful as a biosensor for an analyzing device for POCT.

## Claims

1. A method of manufacturing a biosensor in which a liquid specimen is developed to a development layer by using chromatography and a test substance in the liquid specimen is measured, wherein a required amount of the liquid specimen is reduced by reducing a thickness of the development layer.

2. A biosensor in which a liquid specimen is developed to a development layer by using chromatography and a test substance in the liquid specimen is measured, wherein the development layer is 20 µm to 135 µm in thickness.

3. The biosensor according to claim 2, further comprising:
a support that supports the development layer;
a specimen adding portion for adding the liquid specimen to the development layer;
a labeled reagent portion that reacts with a test substance in the liquid specimen; and
reagent immobilizing portions each of which contains an immobilized reagent specifically reacting with one of the test substance and a labeled reagent.

4. The biosensor according to claim 2, wherein the development layer is formed of at least one layer.

5. The biosensor according to claim 2, wherein the biosensor is composed of an immuno-chromatographic sensor.

6. The biosensor according to claim 5, wherein the biosensor is composed of an immuno-chromatographic sensor for one-step measurement.

7. The biosensor according to claim 2, wherein the development layer is composed of a porous film.
